# EUROPEAN PATENT APPLICATION

(11) **EP 4 600 642 A1**
(43) Date of publication of application: **13.08.2025**
(21) Application number: 23874909.7
(22) Date of filing: 04.10.2023
(51) Int. Cl.: G01N 31/00, A61K 47/00, G01N 33/15, G01N 33/50, C07C 53/126, C07D 291/06

(54) **METHOD FOR SELECTING ADDITIVE CAPABLE OF INHIBITING NITROSATION REACTION, AND PREPARATION PREVENTED FROM GENERATION OF NITROSO COMPOUND**

(30) Priority: 05.10.2022 US 202263413310 P
(71) Applicant: Sawai Pharmaceutical Co., Ltd., Yodogawa-ku Osaka-shi Osaka 532-0003 (JP)
(72) Inventor: YAMAMOTO Hiroyuki, Osaka City, Osaka 532-0003 (JP); TAKEGAWA Yuuki, Osaka City, Osaka 532-0003 (JP); MASUI Tatsuma, Osaka City, Osaka 532-0003 (JP); MINAMIOKA Saki, Osaka City, Osaka 532-0003 (JP)
(74) Representative: Stolmár & Partner Patentanwälte PartG mbB
(86) International application number: PCT/JP2023/036247
(87) International publication number: WO 2024/075784

(57) **Abstract**

One embodiment according to this invention provides a method for selecting an additive agent for suppressing a nitrosation reaction. Alternatively, one embodiment of the present invention provides a formulation that suppresses the generation of nitroso compounds, including an additive agent selected by a method for selecting an additive agent for suppressing a nitrosation reaction. In one embodiment, a method for selecting an additive agent for suppressing a nitrosation reaction includes adding an amine source and an NOx source to an additive agent to generate nitroso compounds, quantifying an amount of the generated nitroso compounds, and evaluating a suppressing effect of the nitrosation reaction of the additive agent based on the amount of the generated nitroso compounds.

## Description

### TECHNICAL FIELD

One embodiment of the present invention relates to a method for selecting an additive agent for suppressing a nitrosation reaction, and in particular, to a method for selecting an additive agent for suppressing a nitrosation reaction in a pharmaceutical composition or formulation for pharmaceutical applications. Alternatively, an embodiment of the present invention relates to a formulation which contains an additive agent for suppressing a nitrosation reaction and suppresses the generation of nitroso compounds.

### BACKGROUND ART

Nitroso compounds are a generic term for organic compounds with nitroso groups, and nitrosamine is a type of nitroso compound generated by nitrosation (addition of a nitroso group on the amine nitrogen) of amines by a NOx source such as nitrous acid. In recent years, the inclusion of nitrosamines suspected to be carcinogenic in some pharmaceutical preparations has triggered the need for risk assessment and reduction measures for nitroso compounds in the development of pharmaceutical preparations. An acceptable limit value for nitroso compounds are defined by notifications issued by regulatory authorities (for example, Voluntary Inspection on the Risk of Nitrosamines Contamination in Pharmaceutical Preparations dated October 8, 2021).

Since active pharmaceutical ingredients often have an amine structure, it is not practical to eliminate amines from the formulation and control the amount of generated nitrosamines. In addition, since NOx sources are contained in trace amounts in the water used in manufacturing, in the air, and in additive agents, it is also impractical to control the amount of generated nitrosamines by blocking all NOx sources.

Although it has been suggested that nitrosation reactions can be suppressed by antioxidants (Non Patent Literature 2), antioxidants cannot be used if they accelerate the degradation of the active ingredient or cause the elution behavior to deviate from the target profile. Therefore, a new additive agent for suppressing a nitrosation reaction was desired as an alternative to antioxidants.

### CITATION LIST

### NON PATENT LITERATURE

Non Patent Literature 1: Voluntary Inspection on Risk of Nitrosamines Contamination in Pharmaceutical Preparations dated October 8, 2021
Non Patent Literature 2: Nanda, K. et al. Inhibition of N-Nitrosamine Formation in Drug Product: A Model Study, J. Pharm. Sci.2021; 110: 3773-3775.

### SUMMARY OF INVENTION

### TECHNICAL PROBLEM

One object of the present invention is to provide a method for selecting an additive agent for suppressing a nitrosation reaction.

Alternatively, one object of the present invention is to provide a formulation which contains an additive agent selected based on the method for selecting the additive agent for suppressing a nitrosation reaction and suppresses the generation of nitroso compounds.

### SOLUTION TO PROBLEM

According to one embodiment of the invention, there is provided a method for selecting an additive agent for suppressing a nitrosation reaction, including adding an amine source and an NOx source to an additive agent to generate a nitroso compound quantifying an amount of the generated nitroso compound, and evaluating a suppressing effect of the nitrosation reaction of the additive agent based on an amount of the generated nitroso compound.

The additive agent with the amine source and the NOx source may be heated under a sealed condition to generate nitroso compounds.

The amine source may be a secondary amine, a tertiary amine, or a quaternary amine.

The NOx source may be selected from a group consisting of nitrous acid, nitrite ion, nitrite salt, nitric acid, nitrate salt, nitrous acidium ion, nitrite esters, peroxynitrite salt, nitrosonium ion, nitro compounds, anhydrous nitrous acid, dinitrogen tetroxide, nitrosyl halides, nitrosyl thiocyanate, nitrosophenols, nitrosothiol, aqua regia, nitric oxide, nitrogen dioxide, and nitrile chloride.

According to one embodiment of the invention, there is provided a formulation containing one or more additive agents selected by the method for selecting an additive agent for suppressing a nitrosation reaction according to any of the above.

One or more additive agents may be selected from a group consisting of sodium stearyl fumarate, magnesium oxide, dry methacrylic acid copolymer LD, macrogol 6000, pregelatinized starch, carmellose, aspartame, crospovidone, microcrystalline cellulose, acesulfame potassium, calcium stearate, Kollicoat IR, polyvinyl alcohol, croscarmellose sodium, sodium starch glycolate, carmellose calcium, magnesium stearate, and hydroxypropyl cellulose.

One or more additive agents may be selected from a group consisting of sodium stearyl fumarate, magnesium oxide, dry methacrylic acid copolymer LD, macrogol 6000, pregelatinized starch, carmellose, aspartame, crospovidone, microcrystalline cellulose, and acesulfame potassium.

One or more additive agents may be selected from a group consisting of magnesium oxide, dry methacrylate copolymer LD, pregelatinized starch, carmellose, and aspartame.

### ADVANTAGEOUS EFFECTS OF INVENTION

According to one embodiment of the present invention, there is provided a method for selecting an additive agent for suppressing a nitrosation reaction. Alternatively, according to one embodiment of the present invention, there is provided a formulation which contains an additive agent selected based on the method for selecting an additive agent suppressing a nitrosation reaction and suppresses the generation of nitroso compounds.

### BRIEF DESCRIPTION OF DRAWINGS

FIG. 1 is a flow diagram showing a method for selecting an additive agent for suppressing a nitrosation reaction according to one embodiment of the invention.
FIG. 2 shows amounts of NDLRT generated by formulations of Example 33, Comparative example 3, and Reference example 5 stored at 40°C and 75% RH for 4 weeks.
FIG. 3 shows amounts of NDLRT generated by formulations of Example 33, Comparative example 3, and Reference example 5 stored at 25°C and 55% RH for 3 weeks.

### DESCRIPTION OF EMBODIMENTS

A method for selecting an additive agent for suppressing a nitrosation reaction and a formulation suppressing the generation of nitrosamines will be described below with reference to the drawings. In addition, the method for selecting an additive agent for suppressing a nitrosation reaction and the formulation suppressing the generation of nitrosamines are not to be interpreted as limited to the contents described in the following Embodiments and Examples. In addition, in the drawings referred to in this embodiment and the examples described below, identical parts or parts having similar functions are marked with the same symbol, and repeated descriptions thereof are omitted.

In this description, "nitroso compound" is a generic term for compounds having a nitroso group represented by a Chemical Formula 1 below. In other words, "nitroso compound" is a generic term for compounds having a structure in which a nitroso group is attached to the nitrogen of amine, amide and the like. As a nitroso compound, although N-nitrosonornicotine (NNN), 4-(methylnitrosamino)-1-(3-pyridyl)-1-butanone (NNK), 4-(methylnitrosamino)-1-(3-pyridyl)-1-butanol (NNAL), N,N'-dinitrosopentamethylenetetramine (DPT), N-nitrosomethylethylamine (NMEA), N- nitrosopiperidine (NPIP), N-nitrosopyrrolidine (NPYR), N-nitrosomorpholine (NMOR), N-nitrosoproline (NPRO), N-nitrososarcosine (NSAR), N-nitrosodimethylamine (NDMA), N-nitrosodiethylamine (NDEA), N nitrosomethylphenylamine (NMPA), N-nitrosodiisopropylamine (NDIPA), N-nitrosoethylisopropylamine (NEIPA), N N-nitrosodibutylamine (NDBA), and N-nitrosomethylaminobutyric acid (NMBA) can be use as examples, the nitroso compounds are not limited to these.

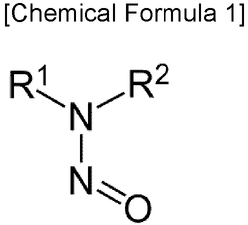

In particular, NDMA, NDEA, NMPA, NDIPA, NEIPA, NDBA, and NMBA are nitroso compounds that may be present as impurities in active pharmaceutical ingredients and pharmaceutical preparations listed in Control of Nitrosamine Impurities in Human Drugs, Guidance for Industry, February 2021, Revision 1.

In this specification, "NOx source" is a generic term for compounds containing nitroso groups capable of generating nitroso compounds by a reaction with an amine source. In addition, NOx is synonymous with nitrogen oxide. As NOx sources, in addition to nitrite, nitric ion and nitrite salts, although nitric acid and nitrates, nitrous acidium ion, nitrite esters, peroxynitrite, nitrosonium ion, nitro compounds, nitric anhydride, dinitrogen tetroxide, nitrosyl halides, nitrosyl thiocyanate, nitrosophenols, nitrosothiols, aqua regia, nitric oxide, nitrogen dioxide, and nitrile chloride can be use as examples, the NOx sources are not limited to those.

In this specification, "amine source" is a generic term for amine compounds capable of generating nitroso compounds by a reaction with NOx sources. Further, amine sources also include compounds that nitrosate, such as urea and amide compounds. In an embodiment, secondary, tertiary, or quaternary amines can be used as amine sources. Since reactivity to the NOx source differs depending on the amine source, it is preferable to use an amine source that contributes to the generation of the nitroso compound to be measured. In addition, in one embodiment, from the viewpoint of suppressing the generation of nitroso compounds in pharmaceutical compositions or formulations, more accurate selection of additives that suppress the nitrosation reaction of an active pharmaceutical ingredient can be achieved if an active pharmaceutical ingredient to be added to them is used as an amine source.

The inventors have found that since active pharmaceutical ingredients having a secondary amine or tertiary amine skeletons in their structure may generate nitroso compounds during the formulation process, it is possible to reduce an amount of nitroso compounds in a pharmaceutical composition or formulation by using an additive agent suppressing nitrosation reactions in combination with the active pharmaceutical ingredient.

### [Method for Selecting Additive Agent for Suppressing Nitrosation Reaction]

In this embodiment, a pharmaceutically acceptable additive agent is used as a measurement sample. The present invention is not limited to this, and can also be used as a method for evaluating the suppressing effects of nitrosation reactions on food additives.

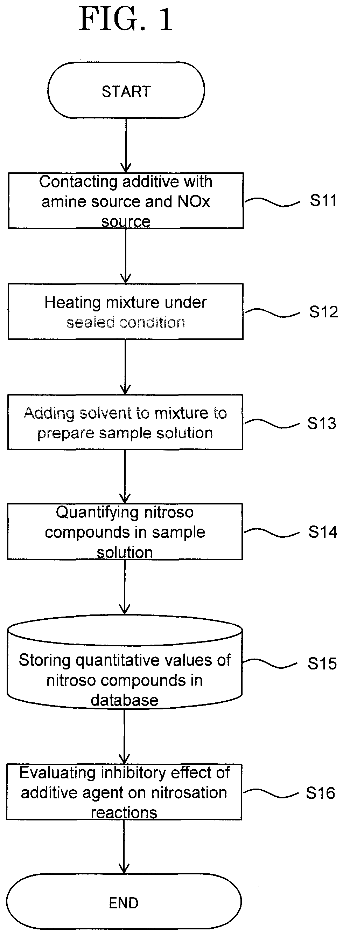

FIG. 1 shows a flow diagram of the method for selecting an additive agent for suppressing a nitrosation reaction according to this embodiment. In the method for selecting an additive agent for suppressing a nitrosation reaction according to this embodiment, the additive agent is contacted with an amine source and a NOx source to generate nitroso compounds (Step S11). In one embodiment, the additive agent to be evaluated, the amine source, and the NOx source are added to a sealable container and mixed, and the amine source and NOx source are contacted together with the additive agent.

The additive agent in contact with the amine source and the NOx source is heated under a sealed condition to generate nitroso compounds (Step S12). The term "sealed condition" indicates a state in which a mixture thereof is sealed in a hermetically sealed container, or a state in which the container containing the mixture thereof is tightly closed.

In this embodiment, the mixture should be heated to 40°C for the purpose of promoting nitrosation of the amine source by the NOx source. In one embodiment, the mixture of an additive agent, amine source, and NOx source is stored under the sealed condition in a thermostatic chamber at 40°C and 75% relative humidity (75% RH). A storage period is, for example, 2 days, 3 days, 1 week, 2 weeks, or 4 weeks.

An appropriate solvent is added to the mixture after a specified period of storage to extract the nitroso compounds. Solid components such as additive agents are removed from the extracted solution using a solid-liquid separation means such as a filter to obtain a sample solution (Step S13). The solvent can be appropriately selected from solvents that can dissolve the nitroso compounds and do not affect quantitation of the nitroso compounds. In one embodiment, a water/methanol mixture (7:3) can be used.

The nitroso compounds are quantified in the sample solution (Step S14). In one embodiment, a liquid chromatography tandem mass spectrometer (LC-MS/MS) may be used to quantify the nitroso compounds. In addition, in this embodiment, the method for quantifying nitroso compounds is not limited to the LC-MS/MS.

The quantified amount of nitroso compounds generated can be associated with the additive agent used and stored in a database (Step S15).

The suppressing effect of nitroso compound generation among each additive is evaluated by comparing the amount of generated nitroso compounds (Step S16).

### [Method for Considering Formulations with Reduced Nitroso Compounds]

Using one or more additive agents selected from the additive agents selected by the method for selecting an additive agent for suppressing a nitrosation reaction described above, it is possible to consider the formula in which the amount of nitroso compounds in the pharmaceutical composition or formulation is below the acceptable limit value. In one embodiment, one or more additive agents can be selected from the selected additive agents for the active pharmaceutical ingredient so that the amount of the nitroso compounds in the pharmaceutical composition or formulation is below the acceptable limit value, and the formula of the pharmaceutical composition or formulation can be determined by adjusting the amount of the selected additive agents.

In more detail, in this embodiment, the type and amount of additive agents to be added to the pharmaceutical composition or formulation are selected from pharmaceutically acceptable additive agents suitable for achieving quality standards required for pharmaceutical preparations, such as dissolution behavior or chemical stability of the active pharmaceutical ingredient, or disintegrability or hardness of a tablet, using the amount of nitroso compounds generated as an indicator, thereby making it possible to reduce the amount of nitroso compounds in the pharmaceutical composition or formulation to be below the acceptable limit value.

Further, in one embodiment, the antioxidant is known to suppress the generation of nitroso compounds and can be used in combination with one or more additive agents selected by the method for selecting an additive agent for suppressing a nitrosation reaction.

Conventionally, for example, although the dissolution behavior or chemical stability of the active pharmaceutical ingredient or the disintegrability or hardness of the tablet has been controlled by adjusting the type and amount of additive agents used in the tablet, it has been difficult to control the amount of nitroso compounds generated in the tablet. By using the method for considering formula according to this embodiment, the amount of nitroso compounds generated in the pharmaceutical composition or formulation can be kept below the acceptable limit value while obtaining the desired properties.

### [Additive Agent Capable of Reducing Generation of Nitroso Compounds]

As additive agents capable of reducing the generation of nitroso compounds, additive agents selected from a group consisting of sodium stearyl fumarate, magnesium oxide, dry methacrylic acid copolymer LD, macrogol 6000, pregelatinized starch, carmellose, aspartame, crospovidone, microcrystal cellulose, acesulfame potassium, calcium stearate, Kollicoat IR, polyvinyl alcohol, croscarmellose sodium, sodium starch glycolate, carmellose calcium, magnesium stearate, and hydroxypropyl cellulose can be exemplified.

Further, in one embodiment, additive agents capable of reducing the generation of nitroso compounds are preferred additive agents selected from a group consisting of sodium stearyl fumarate, magnesium oxide, dry methacrylic acid copolymer LD, macrogol 6000, pregelatinized starch, carmellose, aspartame, crospovidone, microcrystal cellulose, and acesulfame potassium

In addition, in one embodiment, additive agents capable of reducing the generation of nitroso compounds are preferred additive agents selected from a group consisting of magnesium oxide, dry methacrylic acid copolymer LD, pregelatinized starch, carmellose, and aspartame.

### [Formulation that Suppresses Generation of Nitroso Compounds]

By using the method for considering formula according to the embodiment described above, a pharmaceutical composition or formulation in which the generation of nitroso compounds is suppressed can be obtained. In this specification, "pharmaceutical composition" is a mixture of an active pharmaceutical ingredient and one or more pharmaceutically acceptable additive agents. In this specification, "formulation" indicates a dosage form such as granules or dry syrup made by granulating the pharmaceutical composition, an injection form made by dissolving the pharmaceutical composition in a pharmaceutically acceptable solvent, a tablet made by compression molding the pharmaceutical composition, or a capsule made by sealing the pharmaceutical composition in a capsule. In addition, powders in which the pharmaceutical composition is used as it is are also included in the formulation.

The formulation according to this embodiment includes a pharmaceutical composition containing an active pharmaceutical ingredient and one or more additive agents selected by the method for selecting an additive agent for suppressing a nitrosation reaction. The formulation according to this embodiment includes the active pharmaceutical ingredient and one or more selected additive agents in an amount and combination that is below the acceptable limit value of the nitroso compound.

In addition, in one embodiment, it is also preferable that the solvent used in the manufacture of the pharmaceutical composition or formulation is also selected from the solvents evaluated by the method for selecting an additive agent for suppressing a nitrosation reaction described above, and it is more preferable that the solvent is selected from those that generate the lower amount of nitroso compounds.

In addition, in the case where wet granulation or spray-drying methods are used, nitrosation reactions may be accelerated. In one embodiment, in the case where an additive agent that generates relatively high amounts of nitroso compounds is selected to obtain the desired formulation characteristics, the formulation can also be manufactured by the direct tableting method, in which the active pharmaceutical ingredient and one or more selected additive agents are mixed and compression molded, or by a dry granulation method, for the purpose of controlling the nitrosation reaction.

### [Example]

### [Test Solution]

A citric acid solution was prepared by dissolving 5.25 g of citric acid monohydrate in water to make 1000 ml. 1000 ml of 0.05 mol/l of a disodium hydrogen phosphate reagent was prepared, and an aqueous citric acid solution was added thereto to adjust the pH to 4.0 to obtain a test solution (pH 4.0).

### [Amine Reagent]

2 ml of dimethylamine was accurately taken and a test solution (pH 4.0) was added to make exactly 20 ml to obtain a dimethylamine test solution (960 mmol/l).

### [Nitrite Reagent]

24 mg of sodium nitrite was accurately weighed and a test solution (pH 4.0) was added to make exactly 50 ml. 1 ml of this solution was accurately taken and the test solution (pH 4.0) was added to make exactly 25 ml.

### [Standard Solution]

20 µl of N-nitrosodimethylamine (hereafter also referred to as NDMA) standard (1 mg/ml) was quantified into another measuring flask using a micropipette, and a water/methanol mixture (7:3) was added to make exactly 20 ml to obtain 1000 ng/ml of a NDMA solution. Using a micropipette, 959 µl of the 1000 ng/ml NDMA solution was quantified and a water/methanol mixture (7:3) was added to obtain exactly 100 ml of the standard solution.

### [Examples 1 to 15, Comparative Example 1, and Reference Examples 1 to 2]

In a headspace vial, each additive agent to be measured and lactose hydrate were weighed to 500 mg in the mixture ratio shown in Table 1. 20 µl of the reaction reagent (480 mmol/l of dimethylamine, 137 mmol/l of sodium nitrite, diluted with test solution (pH 4.0)) was added. The headspace vial was tightly closed and stored in a thermo-hygrostat (40°C, 75% RH) for 88 hours. In addition, Reference examples 1 to 2 are antioxidants.

10 ml of a water/methanol mixture (7:3)) was added to the stored sample and shaken with a shaker for 20 minutes. The diluted sample was filtered through a membrane filter with a pore size of 0.45 µm or less, and the filtrate was used as the sample solution.

### [0.1% Formic Acid - 5 mM Ammonium Formate-Containing Buffer]

About 315 mg of ammonium formate was weighed, 1000 ml of water was added to dissolve, and 1 ml of formic acid was added.

### [0.1% Formic Acid - 5 mM Ammonium Formate in Methanol]

About 315 mg of ammonium formate was weighed, 1000 ml of methanol was added to dissolve, and 1 ml of formic acid was added.

### [Quantitation of Nitroso Compounds]

LC/MS/MS: (LC: Nexera (registered trademark) X2 (Shimadzu Corporation),
MS/MS: QTRAP5500 triple quadrupole mass spectrometer (AB SCIEX Corporation))
Column: A stainless steel tube having an inner diameter of 2 mm and a length of 15 cm was packed with fully porous 3 µm silica gel for liquid chromatography modified with an octadecyl group and both ion exchange groups.
Mobile phase A: 0.1% formic acid - 5 mM ammonium formate solution
Mobile phase B: 0.1% formic acid - 5 mM ammonium formate in methanol
Column temperature: Constant temperature around 40°C

The amount of NDMA contained in each sample solution was measured under the measurement conditions described above. The amount of NDMA in the lactose hydrate 200M as a Reference was set as 100%, and a generation ratio of the amount of NDMA contained in each sample solution was calculated. The calculated generation ratios of NDMA for each additive are shown in Table 1. In addition, ND in the table indicates that it is below a detection limit. Further, the detection limit is 0.96 ng/ml.

**[Table 1]**

| | Additive agent^{*1} | NDMA generation ratio (%)^{*2} |
|---|---|---|
| Example 1 | Sodium stearyl fumarate | 60 |
| | (7:3) | |
| Example 2 | Dry methacrylic acid copolymer LD (7:3) | ND |
| Reference Example 1 | Propyl gallate | 10 |
| | (9:1) | |
| Example 3 | Macrogol 6000 | 35 |
| | (7:3) | |
| Reference Example 2 | Ascorbic acid | 2 |
| | (9:1) | |
| Example 4 | Pregelatinized starch | 31 |
| | (1:1) | |
| Example 5 | Carmellose | ND |
| | (1:1) | |
| Example 6 | Aspartame | 3 |
| | (7:3) | |
| Example 7 | Crospovidone | 64 |
| | (1:1) | |
| Example 8 | Microcrystalline cellulose | 36 |
| | (1:1) | |
| Example 9 | Acesulfame-K | 55 |
| | (7:3) | |
| Example 10 | Calcium stearate | 35 |
| | (1:1) | |
| Example 11 | Polyvinyl alcohol | 13 |
| | (1:1) | |
| Example 12 | Croscarmellose sodium | 9 |
| | (1:1) | |
| Example 13 | Sodium starch glycolate | 2 |
| | (1:1) | |
| Example 14 | Carmellose calcium | 7 |
| | (1:1) | |
| Example 15 | Magnesium stearate | 44 |
| | (7:3) | |
| Comparison Example 1 | Hydroxypropyl cellulose | 120 |
| | (1:1) | |

| | | |
|---|---|---|
| *1: Lower indicates a mixing ratio of lactose hydrate: each additive agent. *2: A generation ratio in the case where the amount of NDMA in the lactose hydrate is set to 100 | | |

The results in Table 1 show that the additive agents in the Examples 1 to 15 had a NDMA generation ratio of 100% or less, and as compared with the hydroxypropyl cellulose of the Comparative example 1, the generation of NDMA was significantly suppressed.

### [Examples 16 to 32, Comparative Example 2, and Reference Examples 3 to 4]

In the above examples, additive agents capable of suppressing the generation of nitroso compounds by contact with liquid amines were selected. In Examples 16 to 32 and a Comparative example 2, additive agents capable of suppressing the generation of nitroso compounds by contact with solid amines were selected. Each additive agent to be measured, the amine source (dimethylamine or desloratadine), and the NOx source (dextrin) were mixed in the headspace vial. The headspace vial was tightly closed and stored in a thermo-hygrostat (40°C, 75% RH) for 9 days in the case where dimethylamine was added and 1 month in the case where desloratadine was added.

10 ml of a water/methanol mixture (7:3)) was added to the stored sample and shaken with a shaker for 20 minutes. The diluted sample was filtered through a membrane filter with a pore size of 0.45 µm or less, and the filtrate was used as the sample solution.

### [Standard Solution]

10 mg of N-nitorosodesloratadine was weighed accurately and made up to 50 ml accurately by adding methanol. 5 ml of this solution was quantified accurately and made up to exactly 100 ml by adding a water/methanol mixture (1:1). 18 ng/ml was considered 100%, and standard solutions were prepared at several concentrations from 10% to 479%.

### [Quantitation of Nitroso Compounds]

The amount of nitrosodimethylamine (NDMA) or nitorosodesloratadine (NDLRT) in each sample solution was determined according to the quantitation of the nitroso compounds described above. The amount of NDMA or NDLRT generated in each sample solution is converted per maximum daily dose (MDD) and the results are shown in Table 2.

**[Table 2]**

| | Additive agent | NDMA (ng/MDD) | NDRLT (ng/MDD) |
|---|---|---|---|
| Example 16 | Sodium stearyl fumarate | 83 | 13 |
| Example 17 | Magnesium oxide | 34 | 23 |
| Example 18 | Dry methacrylic acid copolymer LD | 41 | 28 |
| Reference Example 3 | Propyl gallate | 52 | 36 |
| Example 19 | Macrogol 6000 | 105 | 50 |
| Reference Example 4 | Ascorbic acid | 61 | 59 |
| Example 20 | Pregelatinized starch | 18 | 66 |
| Example 21 | Carmellose | 60 | 83 |
| Example 22 | Aspartame | 64 | 96 |
| Example 23 | Crospovidone | 39 | 134 |
| Example 24 | Microcrystalline cellulose | 47 | 150 |
| Example 25 | Acesulfame-K | 92 | 150 |
| Example 26 | Calcium stearate | 80 | 167 |
| Example 27 | Polyvinyl alcohol-polyethylene glycol graft copolymer | 61 | 176 |
| Example 28 | Polyvinyl alcohol | 113 | 202 |
| Example 29 | Croscarmellose sodium | 62 | 206 |
| Example 30 | Sodium starch glycolate | 47 | 225 |
| Example 31 | Carmellose calcium | 56 | 268 |
| Example 32 | Magnesium stearate | 96 | 275 |
| Comparison Example 2 | Hydroxypropyl cellulose | 216 | 319 |

The results in Table 2 show that the additive agents of the Examples 16 to 32 suppressed the generation of nitrosamines better than the additive agent of the Comparative example 2. In addition, the additive agents of examples 16 to 25 suppressed the generation of nitrosamines by more than 50% than the additive agent of the Comparative example 2. Furthermore, the additive agents of the Examples 17, 18, 20, 21, and 22 suppressed nitrosamines by more than 70% than the additive agent of the Comparative example 2. In particular, the suppressing effects of the additive agents of the Examples 17, 18, 20, 21, and 22 on nitrosamine generation was comparable to that of the antioxidants of the Reference examples 3 to 4, indicating that they can be used as nitrosamine generation suppressing additive agents to replace antioxidants.

### [Example 33, Comparative Example 3, and Reference Example 5]

In the above examples, although the suppressing effect of individual additive agents on nitrosamine generation was evaluated, in Example 33, Comparative Example 3, and Reference Example 5, the suppressing effects of nitrosamine generation in the formulation was evaluated. Desloratadine and various additives were weighed, mixed, and sieved to obtain a mixture so that the amount in one tablet was as shown in Table 3 below. Tablets (<p10.0, 2-step R-face: R=11.5, r=4) were made using a tableting machine (TK-HP20KN, Tokushu Keisoku Co., Ltd.) so that each tablet weighed approximately 550 mg.

**[Table 3]**

| constituent name | Reference Example 5 | Example 33 | Comparison Example 3 |
|---|---|---|---|
| Desloratadine | 5 | 5 | 5 |
| Dilactose | 439.5 | 439.5 | 439.5 |
| Hydroxypropyl cellulose | 25 | 25 | 25 |
| Crospovidone | 25 | 25 | 25 |
| Magnesium stearate | 5 | 5 | 5 |
| Yellow ferric oxide | 0.5 | 0.5 | 0.5 |
| L-Ascorbic acid | 2 | - | - |
| Carmellose | - | 2 | - |
| Magnesium metasilicate aluminate | - | - | 2 |
| Total weight (mg/Tab) | 501.7 | 501.7 | 501.7 |

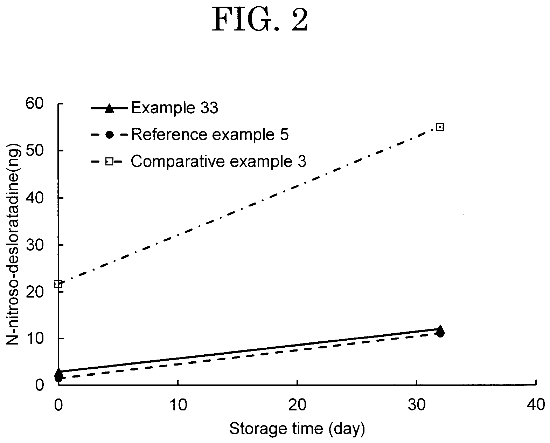

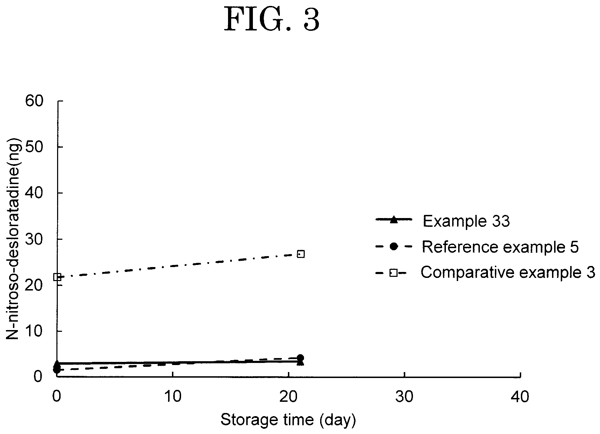

The tablets of the Example 33, the Comparative example 3, and the Reference example 5 were tightly closed and stored in a thermo-hygrostat at 25°C and 55% RH for 3 weeks or at 40°C and 75% RH for 4 weeks. The amount of nitorosodesloratadine (NDLRT) in the tablets of the Example 33, the Comparative example 3, and the Reference example 5 after storage was determined by the method for quantitation of nitroso compounds described above. The results of storing the formulations of the Example 33, the Comparative example 3, and the Reference example 5 at 40°C and 75% RH for 4 weeks are shown in FIG. 2, and the results of storing them at 25°C and 55% RH for 3 weeks are shown in FIG. 3.

As shown in the results in FIG. 2 and FIG. 3, carmellose, in which the suppressing effect of nitrosation reactions was found in the Examples 5 and 21, was as effective as the antioxidant in the Reference example 5 in the suppressing effect of the nitrosation reactions in the formulation.

## Claims

1. A method for selecting an additive agent for suppressing a nitrosation reaction comprising:
adding an amine source and an NOx source to an additive agent to generate a nitroso compound;
quantifying an amount of the generated nitroso compound; and
evaluating a suppressing effect of the nitrosation reaction of the additive agent based on an amount of the generated nitroso compound.

2. The method for selecting the additive agent for suppressing the nitrosation reaction according to claim 1, wherein the additive agent added with the amine source and the NOx source is heated under a sealed condition to generate the nitroso compound.

3. The method for selecting an additive agent for suppressing a nitrosation reaction according to claim 1, wherein the amine source is a secondary amine, a tertiary amine, or a quaternary amine.

4. The method for selecting the additive agent for suppressing the nitrosation reaction according to claim 1, wherein the NOx source is selected from a group consisting of nitrous acid, nitrite ion, nitrite salt, nitric acid, nitrate salt, nitrous acidium ion, nitrite esters, peroxynitrite salt, nitrosonium ion, nitro compounds, anhydrous nitrous acid, dinitrogen tetroxide, nitrosyl halides, nitrosyl thiocyanate, nitrosophenols, nitrosothiol, aqua regia, nitric oxide, nitrogen dioxide, and nitrile chloride.

5. A formulation comprising: one or more additive agents selected by the method for selecting an additive agent for suppressing a nitrosation reaction according to any one of claims 1 to 4.

6. The formulation according to claim 5, wherein the one or more additive agents are selected from a group consisting of sodium stearyl fumarate, magnesium oxide, dry methacrylic acid copolymer LD, macrogol 6000, pregelatinized starch, carmellose, aspartame, crospovidone, microcrystalline cellulose, acesulfame potassium, calcium stearate, Kollicoat IR, polyvinyl alcohol, croscarmellose sodium, sodium starch glycolate, carmellose calcium, magnesium stearate, and hydroxypropyl cellulose.

7. The formulation according to claim 5, wherein the one or more additive agents are selected from a group consisting of sodium stearyl fumarate, magnesium oxide, dry methacrylic acid copolymer LD, macrogol 6000, pregelatinized starch, carmellose, aspartame, crospovidone, microcrystalline cellulose, and acesulfame potassium.

8. The formulation according to claim 5, wherein the one or more additive agents are selected from a group consisting of magnesium oxide, dry methacrylic acid copolymer LD, pregelatinized starch, carmellose, and aspartame.
